# FASCICULE DE BREVET EUROPEEN

(11) **EP 2 020 410 B1**
(45) Date de publication et mention de la délivrance du brevet: **18.12.2013**
(21) Numéro de dépôt: 08017944.3
(22) Date de dépôt: 29.06.2006
(51) Int. Cl.: C07D 471/04, A61K 31/519, A61P 35/00

(54) **Dérivés de pyrido[2,3-d] pyrimidine, leur préparation, leur application en thérapeutique**
Pyrido[2,3-d]pyrimidinderivate,Verfahren zu deren Herstellung und ihre therapeutische Verwendung
Pyrido[2,3-d]pyrimidine derivatives, process for their preparation and their therapeutical use

(30) Priorité: 01.07.2005 FR 0507032
(43) Date de publication de la demande: 04.02.2009
(62) Demande divisionnaire de: 06778710.1
(73) Titulaire: SANOFI, 75008 Paris (FR)
(72) Inventeur: Bourrie, Bernard, 34980 Saint Gely du Fesc (FR); Casellas, Pierre, 34090 Montpellier (FR); Jegham, Samir, 34980 Montpellier (FR); Muneaux, Claude, 34270 Les Matelles (FR); Perreaut, Pierre, 34980 Saint Clément de Rivière (FR)
(74) Mandataire: Jacobson, Claude

(56) Documents cités:
- EP-B- 0 790 997
- WO-A-01/55147
- WO-A-01/70741
- WO-A-03/000011
- WO-A-2004/085436
- SCHROEDER M C ET AL: "SOLUBLE 2-SUBSTITUTED AMINOPYRIDOÄ2,3-DÜPYRIMIDIN-7-YL UREAS. STRUCTURE-ACTIVITY RELATIONSHIPS AGAINST SELECTED TYROSINE KINASES AND EXPLORATION OF IN VITRO AND IN VIVO ANTICANCER ACTIVITY" 7 juin 2001 (2001-06-07), JOURNAL OF MEDICINAL CHEMISTRY, AMERICAN CHEMICAL SOCIETY. WASHINGTON, US, PAGE(S) 1915-1926 , XP001152609 ISSN: 0022-2623 * le document en entier *
- ANDREW M. THOMPSON ET AL.: "Synthesis and Structure-Activity Relationships of soluble 7-Substituted 3-(3,5-Dimethoxyphenyl)-1,6-naphthyridin-2 -amines and Related Ureas as Dual Inhibitors of the Fibroplast Growth Factor Receptor-1 and Vascular Endothelial growth factor Receptor-2 Tyrosine Kinasess" JOURNAL OF MEDICINAL CHEMISTRY., vol. 48, no. 14, 17 juin 2005 (2005-06-17), pages 4628-4653-, XP002372558 USAMERICAN CHEMICAL SOCIETY.

## Description

La présente invention a pour objet des dérivés de pyrido[2,3-d]pyrimidine, leur préparation et leur application en thérapeutique.

Des composés dérivés de pyrido[2,3-d]pyrimidine sont décrits dans les demandes de brevets WO 01/55 147 et WO 03/000 011 et dans les brevets EP-B-790 997 et US 5 733 913. Ces composés sont potentiellement utiles pour traiter les troubles de la prolifération cellulaire.

Ainsi, et selon un premier aspect, la présente invention a pour objet des composés répondant à la formule (I) : 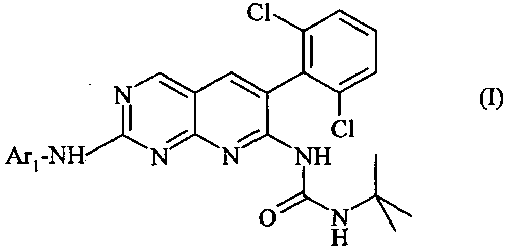 dans laquelle Ar₁ est choisi parmi :

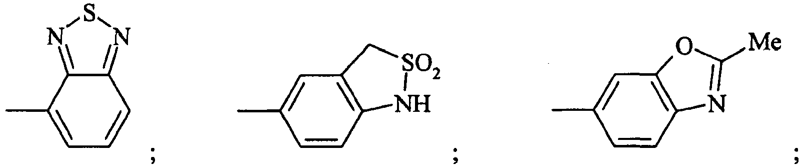

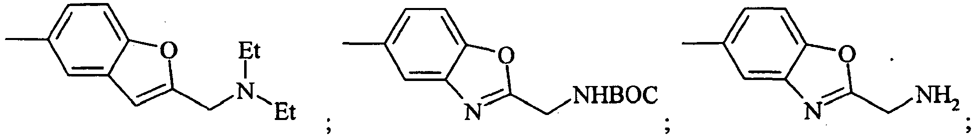

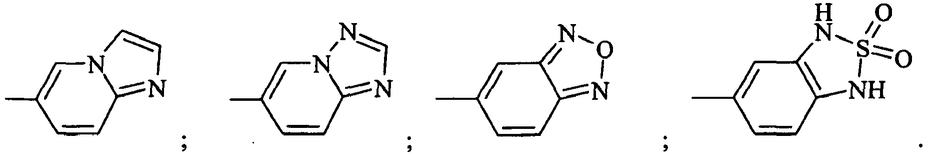

Les composés des exemples qui suivent sont un objet de la présente invention.

Un composé conforme à l'invention peut (i) être sous forme non chirale, ou racémique, ou enrichie en un stéréo-isomère, ou enrichie en un énantiomère ; (ii) être éventuellement salifié, et (iii) être éventuellement hydraté ou solvaté.

Les composés de formule (I) peuvent comporter un ou plusieurs atomes de carbone asymétrique. Ils peuvent donc exister sous forme d'énantiomères ou de diastéréoisomères. Ces énantiomères, diastéréoisomères ainsi que leurs mélanges, y compris les mélanges racémiques, font partie de l'invention.

Les composés de formule (I) peuvent exister à l'état de bases ou de sels d'addition à des acides. Lorsque des composés de formule (I) comportent des fonctions acides libres, par exemple carboxylique, sulfonique, phosphonique, ces fonctions acides peuvent être salifiées à l'aide de bases pour former des sels d'addition. De tels sels d'addition font partie de l'invention.

Les sels d'addition à des acides ou à des bases sont avantageusement préparés avec, respectivement, des acides ou des bases pharmaceutiquement acceptables, mais les sels d'autres acides ou de bases utiles, par exemple, pour la purification ou l'isolement des composés de formule (I) font également partie de l'invention.

Les composés de formule (I) peuvent également exister sous forme d'hydrates ou de solvates, à savoir sous forme d'associations ou de combinaisons avec une ou plusieurs molécules d'eau ou avec un solvant. De tels hydrates et solvates font également partie de l'invention.

Dans le cadre de la présente invention, on entend par :
- un atome d'halogène : un fluor, un chlore, un brome ou un iode ;

Les composés de formule (I) sont préparés par réaction entre un composé de formule (II) : 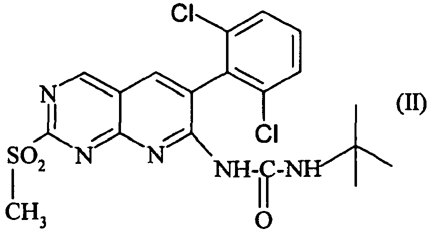 et une amine de formule Ar'₁NH₂ (III) dans laquelle Ar'₁ représente Ar₁, tel que défini pour (I) ou un précurseur de Ar₁; le cas échéant on transforme le groupe Ar'₁ du composé ainsi obtenu en un groupe Ar₁.

Conformément à l'invention, on peut préparer les composés de formule (I) selon un procédé caractérisé en ce que l'on fait réagir :
(i) un composé de formule : 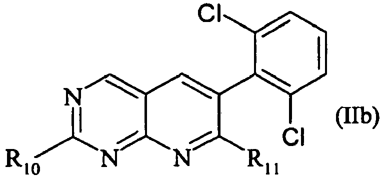 dans laquelle R₁₀ est un groupe partant tel que : (a) halogène, en particulier Cl ou Br, ou (b) alkyl-S(O)m- avec m = 0, 1, ou 2 ; R₁₁ est NHC(O)-NH-tBu; et
(ii) une amine de formule Ar'₁NH₂ (III) dans laquelle Ar'₁ représente Ar₁ tel que défini pour (I) ou un précurseur de Ar₁; le cas échéant on transforme le groupe Ar'₁ du composé ainsi obtenu en un groupe Ar₁.

Lorsque R₁₀ est halogène ou alkyl-S(O)m- avec m = 2, La réaction est effectuée dans un solvant, de préférence polaire :
(i) par exemple le térahydrofurane, le diméthylsulfoxide ou l'éthanol, éventuellement en présence d'une trace d'acide tel que l'acide chlorhydrique ; ou
(ii) dans le diméthylsulfoxide en présence d'une base forte telle que tBuOK ; à une température comprise entre la température ambiante et la température de reflux du solvant.

Lorsque R₁₀ est alkyl-S(O)m- avec m = 0 ou 1, on peut effectuer la réaction avec Ar'₁NH₂ (III) à l'état fondu, de préférence à une température voisine de 200° C, sans catalyseur.

Le cas échéant, les fonctions amines présentes dans le groupe Ar'₁ du composé (III) sont préalablement salifiées ou protégées.

Les composés de formule (II) sont préparés en suivant le mode opératoire décrit dans le brevet européen 790 997 et le brevet US 5 733 913, comme décrit dans le Schéma 1 ci-après :

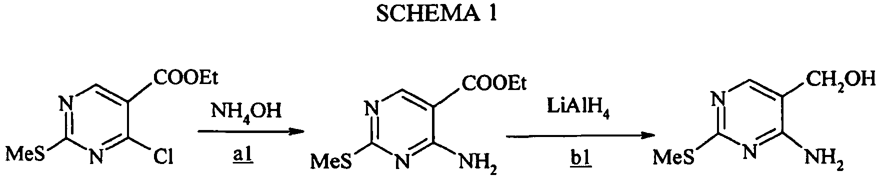

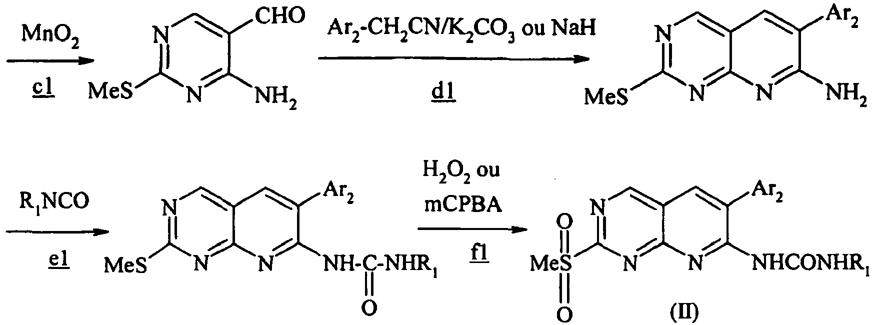

mCPBA : acide *méta*-chloroperbenzoïque.

Les amines de formule (III) Ar'₁NH2 sont connues ou préparées par des méthodes connues à partir des dérivés nitrés Ar'₁NO₂ (IV) correspondants, par réduction soit (i) en milieu acide en présence d'un métal tel que le fer ou le zinc en poudre, soit (ii) par l'hydrogène en présence d'un catalyseur tel que Pd/C. Ar' représente Ar ou un précurseur de Ar.

Les composés de formule (IV) sont connus ou préparés par des méthodes connues.

Les composés selon l'invention sont obtenus sous forme racémique ; on peut ensuite préparer les isomères optiquement purs en utilisant des méthodes de dédoublement connues de l'homme de l'art, telle que la cristallisation par formation de sels avec des agents chiraux. On peut également préparer des composés selon l'invention sous forme optiquement pure en utilisant des méthodes de synthèse asymétrique ou stéréospécifique, l'utilisation de techniques chromatographiques utilisant une phase chirale. Par ailleurs, les produits de l'invention peuvent être séparés *via* la formation de diastéréoisomères, leur séparation, puis la décomposition du diastéréoisomère pharmacologiquement utile en son produit actif énantiomériquement pur. Des techniques enzymatiques peuvent aussi être employées. Des techniques séparatives supplémentaires connues peuvent être utilisées. Elles incluent celles divulguées dans: Enantiomers, Racemates, and Resolutions, John Wiley and Sons, New York (1981). Les composés selon l'invenion peuvent également être préparés sous forme enrichie en un stéréoisomère dès la préparation des intermédiaires de synthèse. Ainsi, le dédoublement des énantiomères des amines de formule (III) ou des précurseurs nitrés (IV) peut être réalisé par une des méthodes précitées.

Les exemples suivants décrivent la préparation de certains intermédiaires et de composés conformes à l'invention. Ces exemples ne sont pas limitatifs et ne font qu'illustrer la présente invention.

Dans les exemples, on utilise les abréviations suivantes :
Boc : *tert*-butoxycarbonyle
BOP : benzotriazol-1-yloxytris(diméthylamino)phosphonium hexafluorophosphate.
THF : tétrahydrofurane
TA : température ambiante
TFA : acide trifluoroacétique
DCM : dichlorométhane
DMSO : diméthylsulfoxyde
DMF: diméthylformamide
MeOH : méthanol.
DCCI : dicyclohexylcarbodiimide
DIPEA :diisopropyléthylamine
KHSO₄ / K₂SO₄ : solution à 5 % de KHSO₄ / K₂SO₄.

Les spectres de résonance magnétique nucléaires (RMN) du proton sont enregistrés à 200 ou 250 MHz dans le DMSO-d₆, sauf indication contraire. Le signal DMSO-d₆ est à 2,5 ppm et sert de référence. Pour l'interprétation des spectres, on utilise les abréviations suivantes : s : singulet, d : doublet, t : triplet, m : massif, mt : multiplet, se : singulet élargi, dd : doublet de doublet, qd : quadruplet, qt : quintuplet.

F : point de Fusion (en degré Celsius) tel que mesuré sur un appareil Büchi B545 avec un gradient de température de 1°C par minute.

MH+ : Spectre de Masse. Les composés sont analysés par couplage HPLC - UV - MS (chromatographie liquide - détection UV - spectrométrie de masse). L'appareil utilisé, commercialisé par Agilent, est composé d'un chromatographe HP 1100 équipé d'un détecteur à barrette de diodes Agilent et d'un spectromètre de masse quadripolaire MSD Quad.

Les conditions analytiques sont les suivantes :
Colonne : Symmetry C18 (50 x 2,1 mm; 3,5 µm)
Eluant A : H₂O + TFA 0,005 % à pH 3,15
Eluant B : CH₃CN + TFA 0,005 %
Gradient :

| Temps (min) | % B |
|---|---|
| 0 | 0 |
| 10 | 90 |
| 15 | 90 |
| 16 | 0 |
| 20 | 0 |

Température de la colonne : 30°C
Débit : 0,4 mL / min
Détection :
λ = 210 nm
tr : temps de rétention
v : volume.

### Préparation d'un composé de formule (II).

### Préparation 1

### N-(t-Butyl)-N'-[6-(2,6-dichlorophényl)-2-(méthylsulfonyl)pyrido[2,3-d]pyrimidin-7-yl]urée.

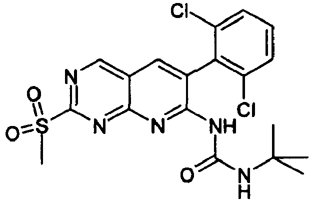

### 1.1 4-Amino-2-(méthylthio)pyrimidine-5-carboxylate d'éthyle.

A une suspension de 50,7 g de 4-chloro-2-(méthylthio)pyrimidin-5-carboxylate d'éthyle dans 400 mL d'EtOH on ajoute en 20 minutes, et en maintenant la température vers 20°C, 140 mL d'une solution NH₄OH à 20%. Après 20 heures d'agitation à température ambiante, le milieu réactionnel est concentré sous vide presque à sec, puis le résidu est repris dans 350 mL d'eau, agité 20 minutes, filtré, lavé avec 3x60 mL d'eau puis est séché sous vide en présence de P₂O₅. On obtient un solide blanc, F = 134-135°C, m = 39,9 g.

### 1.2 [4-Amino-2-(méthylthio)pyrimidin-5-yl]méthanol.

A 39,68 g d'ester obtenu à l'étape précédente dissous dans 1 litre de THF on ajoute en 45 minutes 210 mL d'une solution 1M en LiAlH₄ dans le THF, en maintenant la température inférieure à 30°C. On agite encore 1 heure puis abaisse la température à 5°C et ajoute successivement goutte à goutte 9 mL d'eau, 6,5 mL de soude 5N puis 32 mL d'eau. Après 10 minutes d'agitation, le solide est filtré puis rincé au THF. Le filtrat est concentré sous vide à sec puis le résidu est redissous dans 600 mL de toluène à ébullition, on filtre rapidement à chaud pour éliminer un peu d'insoluble et laisse refroidir une nuit le filtrat. Les cristaux blancs obtenus sont filtrés, lavés avec un peu de toluène puis d'éther et séchés, F = 124-127°C, m = 23,9 g.

### 1.3 4-Amino-2-(méthylthio)pyrimidine-5-carbaldéhyde.

A une suspension de 23,8 g de l'alcool obtenu à l'étape précédente dans 1600 mL de chloroforme, on ajoute en 2 minutes 79,5 g de MnO₂ actif et agite 1 nuit à température ambiante ; le solide est filtré, lavé par 3x75 mL CHCl₃ et le filtrat concentré sous vide à sec ; le résidu solide blanc est repris dans l'éther, filtré, séché, F = 184-186°C, m = 21,05 g.

### 1.4 6-(2,6-Dichlorophényl)-2-(méthylthio)pyrido[2,3-d]pyrimidin-7-amine.

A 21 g de l'aldéhyde obtenu à l'étape précédente, dissous dans 240 mL de DMF et refroidis à 5°C on ajoute en 5 minutes 5,47 g de NaH 60% puis en 20 minutes, par petites fractions, 29,05 g de 2,6-dichlorophénylacétonitrile. L'agitation est poursuivie 30 minutes à 5°C puis une nuit à température ambiante. Le milieu réactionnel est refroidi à 5°C et on ajoute 65 mL d'une solution saturée en NH₄Cl puis 500 mL d'un mélange eau/glace ; il se forme un précipité rouge qui est filtré, lavé 2 fois à l'eau, essoré au maximum, lavé à l'éther, par 100 mL de chloroforme, puis à l'éther à nouveau ; après séchage on obtient un solide beige, F = 250-253°C, m = 29,92 g.

Les phases éther et chloroforme de lavage sont concentrées à sec, on reprend dans un peu de chloroforme auquel on ajoute de l'éther : on obtient un deuxième jet de 3,15 g, m totale= 33,07 g.

### 1.5 N-(t-Butyl)-N'-[6-(2,6-dichlorophényl)-2-(méthylthio)pyrido[2,3-d]pyrimidin-7-yl]urée.

A 29,9 g de l'amine obtenue ci-dessus en solution dans 300 mL de DMF, on ajoute en 10 minutes et en maintenant la température inférieure à 25°C, 4,6 g de NaH à 60% ; on agite encore 20 minutes puis ajoute en 20 minutes 12,2 mL d'isocyanate de tertiobutyle puis agite une nuit. Le milieu réactionnel est versé lentement sur 800 mL d'un mélange eau/glace +100 mL d'HCl 6N ; le précipité formé est filtré, lavé à l'eau, essoré puis agité 1 heure dans 300 mL d'éther, puis filtré, lavé à l'éther et séché. On obtient un solide beige, F = 195-196°C (dec.), m = 26,5 g.

### 1.6 N-(t-Butyl)-N'-[6-(2,6-dichlorophényl)-2-(méthylsulfonyl)pyrido[2,3-d]pyrimidin-7-yl]urée.

A 21,95 g d'urée obtenue ci dessus en solution dans 300 mL de chloroforme, on ajoute en 25 minutes, et en maintenant la température inférieure à 25°C, 27 g d'acide métachloroperbenzoïque (77%). Il se forme un précipité. Après 2 heures le milieu réactionnel est dilué par 1 litre de dichlorométhane et on ajoute du Na₂SO₄, puis 14 g de Ca(OH)₂. Après 30 minutes d'agitation le solide est filtré, lavé par du dichlorométhane puis le filtrat concentré à sec. Le résidu est trituré dans 80 mL d'éther à chaud ; on laisse refroidir, puis le solide blanc est filtré, lavé avec de l'éther et séché, F = 138-140°C, m= 20,5 g.

**RMN: 1,40: s: 9H; 3,50: s: 3H; 7,50-7,70: m: 3H; 8,55: s: 1H; 9,10: s: 1H; 9,60: s: 1H; 9,95: s: 1H.**

### Préparation des composés de formule (III).

Les numéros de préparations utilisés renvoient aux numéros des composés du tableau 2 ci-après.

### Préparation 18

Produit commercial.

### Préparation 19

Préparé selon J. Hetero. Chem. 1986, 23, 1645-1649 et isolé sous forme de chlorhydrate.

### Préparation 20

Préparé selon J. Chem. Soc. 1928, 121.

### Préparation 21

### 21.1

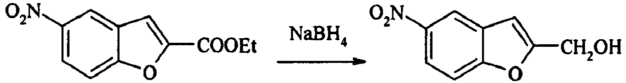

A 1,12 g de 5-nitro-benzo[b]furane-2-carboxylate d'éthyle dans 50 mL de THF, on additionne 2,27 g de NaBH₄ par petites portions sur une durée de 8 heures, puis on agite pendant 40 heures. 5 mL de méthanol puis 5mL d'eau sont ajoutés. Le milieu réactionnel est extrait par AcOEt, la phase organique est lavée par de l'eau, par une solution de KHSO₄/K₂SO₄ 5%, par de l'eau, puis par une solution saturée en NaCl. Après séchage et concentration sous pression réduite, on recueille 0,74 g du produit attendu sous forme solide.

### 21.2

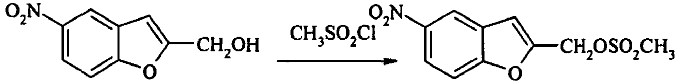

730 mg du produit obtenu à l'étape 21.1 sont dissous dans 9 mL de DCM et maintenus à 5°C. 1 mL de triéthylamine est ajouté à 5°C, puis en 15 minutes, 536 mg de chlorure de méthanesulfonyle sont additionnés. La température est maintenue à 5°C pendant 15 minutes, puis le milieu réactionnel est laissé remonter à températue ambiante pendant 55 minutes. Le milieu réactionnel est ensuite dilué par du DCM et de l'eau. La phase organique est décantée, lavée par de l'eau, par une solution saturée en NaCl, séchée et évaporée sous pression réduite. On obtient 0,98 g d'huile, comprenant un mélange de mésylate (produit attendu) et de chlorure (produit de substitution du CH₂OH en position 2 du benzo[*b*]furane par un CH₂Cl).

### 21.3

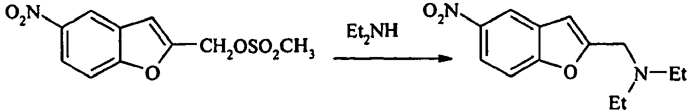

0,97 g du produit obtenu à l'étape 21.2 dans 10 mL de DMF, sont traités par 1,05 g de diéthylamine pendant 18 heures. Le milieu réactionnel est extrait par AcOEt, la phase organique est lavée par de l'eau, par une solution saturée en NaCl, séchée, puis les solvants sont évaporés sous pression réduite. On obtient 0,93 g d'huile.

### 21.4

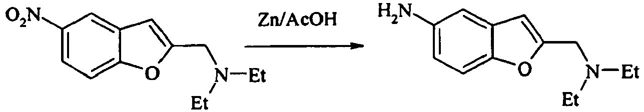

A 1,16 g de produit obtenu à l'étape 21.3 dans 40 mL de THF, on ajoute 4,48 g de Zn en poudre, puis, à -5°C, 5 mL d'acide acétique, sur une période de 25 minutes. Après 1h15 de réaction, le solide résiduel est éliminé du milieu réactionnel par filtration, le solide est lavé par un peu de THF, les phases organiques sont rassemblées, diluées par AcOEt et de l'eau, puis amenées à pH = 9 par NaOH 10N. Après décantation, la phase organique est isolée et lavée par une solution de Na₂CO₃ à 15%, par de l'eau, par une solution saturée en NaCl, séchée et évaporée. On obtient 900 mg d'huile.

### Préparation 22

### 22.1

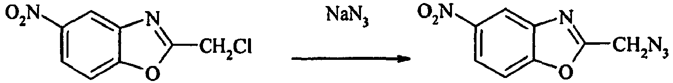

A 1,64 g de 2-chlorométhyl-5-nitro-benzoxazole (préparé selon *Synth*. *Communications* 1989, **19**, 2921-2924) dans 25 mL de DMF, on ajoute 1,26 g d'azoture de sodium et on agite une nuit à température ambiante. Le milieu réactionnel est versé sur 150 mL d'AcOEt et lavé deux fois par de l'eau glacée, puis par une solution saturée en NaCl. La phase organique est séchée et concentrée sous pression réduite. On recueille 1,42 g d'huile noire.

### 22.2

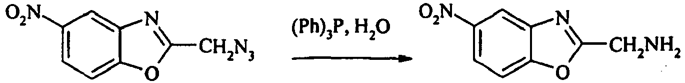

A 1,40 g du produit obtenu à l'étape 22.1 dans 30 mL d'AcOEt, on ajoute 2,84 g de triphénylphosphine en 10 minutes, puis, après 10 minutes, 1,16 mL d'eau sont ajoutés en 2 minutes. Après 24 heures sous agitation à 60°C, puis refroidissement, le milieu réactionnel est dilué par AcOEt, la phase organique est lavée à l'eau puis par une solution saturée en NaCl. La phase organique est séchée et concentrée sous pression réduite. Le résidu est repris dans Et₂O et est extrait deux fois par HCl 1N. Les phases acides sont rassemblées, mises au contact d'AcOEt et amenées à pH = 10 par NaOH 10N. Après décantation, la phase organique est lavée par de l'eau, puis par une solution saturée en NaCl. La phase organique est séchée et concentrée sous pression réduite pour fournir 468 mg du produit attendu sous forme d'huile.

### 22.3

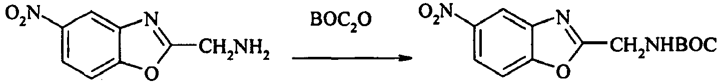

Le produit obtenu à l'étape 22.2 est dissous dans 10 mL de DCM, puis on ajoute 0,4 équivalent de triéthylamine puis 1,1 équivalent de BOC₂O. Après 5H, le milieu réactionnel est dilué par CH₂Cl₂ puis est lavé par, successivement, une solution à 5% KHSO₄/K2SO₄, de l'eau, une solution saturée en NaCl. Le brut est séché et évaporé sous pression réduite pour l'obtention de 388 mg du produit attendu.

### 22.4

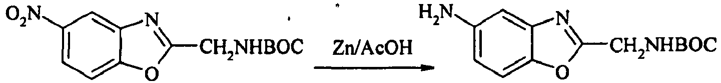

Le produit obtenu à l'étape 19.3 est réduit quantitativement par Zn/AcOH pour l'obtention d'une huile, selon la méthode décrite à la préparation 18.4

### Préparation 23

### 23.1

Préparé selon J. Hetero. Chem. 1973, 10, 755.

### 23.2

Réduction du produit obtenu à l'étape 23.1 par Sn/HBr dans l'eau selon Chem.Abstr. 1950, 4474.

### Préparation 24

Préparé selon J. Hetero. Chem. 1970, 7, 1019-1027.

### Préparation 25

Préparé selon Boll. Sci. Fac. Chim.Ind Bologna 1964 vol.22 pages 33-37

### Préparation 26

Préparé selon le mode opératoire décrit dans la demande de brevet WO92/05164.

Les composés de formule (III) sont caractérisés dans le tableau 2 suivant :

**TABLEAU 2**

| Préparations des composés de formule (III). | | | |
|---|---|---|---|
| Prép. | Ar₁ | X = NO₂ (IV), HCl RMN | X = NH₂ (III) RMN |
| 18 | 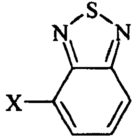 | - | Produit commercial |
| 19 | 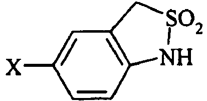 | - | Préparé selon J. Hetero. Chem. 1986, 23, 1645-1649 et isolé sous forme de chlorhydrate |
| 20 | 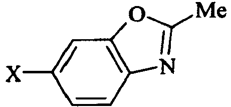 | - | Préparé selon J. Chem. Soc. 1928, 121 |
| 21 | 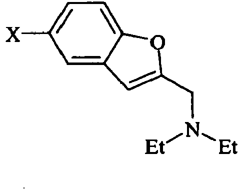 | 21.3 | 21.4 |
| | | 1,00 ppm : t : 6H ; 2,50 ppm : qd : 4H ; 3,75 ppm : s : 2H ; 7,00 ppm : s : 1H ; 7,75 ppm : d : 1H ; 8,15 ppm : d : 1H ; 8,50 ppm : d : 1H. | 1,00 ppm : t : 6H ; 2,55 ppm : qd : 4H ; 3,65 ppm : s : 2H ; 4,90 ppm : se : 2H ; 6,45 ppm : s : 1H ; 6,50 ppm : dd : 1H ; 6,65 ppm : d: 1H; 7,15 ppm: d : 1H. |
| 22 | 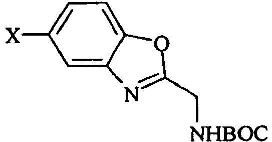 | 22.3 | 22.4 |
| | | 1,40 ppm : s : 9H ; 4,45 ppm : d : 2H ; 7,65 ppm : t : 1H ; 7,95 ppm : d : 1H ; 8,30 ppm : dd : 1H ; 8,60 ppm : d : 1H. | 1,35 ppm : s : 9H ; 4,25 ppm : d : 2H ; 4,95 : se : 2H ; 6,55 ppm : dd : 1H ; 6,75 ppm : d : 1H ; 7,25 ppm : d : 1H ; 7,45 ppm : t : 1H. |
| 23 | 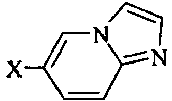 | 23.1 | 23.2 |
| | | Préparé selon J. Hetero. Chem. 1973, 10, 755 | Préparé selon Chem.Abstr. 1950, 4474. |
| 24 | 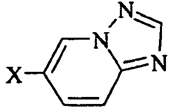 | - | Préparé selon J. Hetero. Chem. 1970, 7, 1019-1027 |
| 25 | 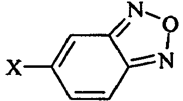 | - | Préparé selon Boll. Sci. Fac. Chim.Ind. Bologna 1964 vol.22 pages 33-37 |
| 26 | 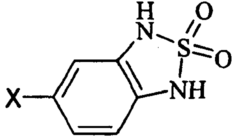 | - | Préparé selon le mode opératoire décrit dans la demande de brevet WO92/05164 |

Les numéros des composés exemplifiés renvoient à ceux donnés dans le Tableau 3 ci-après qui illustre les structures chimiques et les propriétés physiques de quelques composés selon l'invention. Lorsqu'ils contiennent un carbone asymétrique, ces composés sont obtenus sous forme racémique.

### EXEMPLE 1 : (Composé N° 2) N-[2-(2,1,3-benzothiadiazol-4-ylamino)-6-(2,6-dichlorophényl)pyrido[2,3-d]pyrimidin-7-yl]-N'-(1,1-diméthyléthyl)-urée.

Le composé 2 est préparé de la même manière que le composé 1, au départ de l'amine de la préparation 18 du tableau 2.

### EXEMPLE 2 : (Composé N° 3) N-[6-(2,6-dichlorophényl)-2-[(1,3-dihydro-2,2-dioxido-2,1-benzisothiazol-5-yl)amino]pyrido[2,3-d]pyrimidin-7-yl]-N'-(1,1-diméthyléthyl)-urée.

Un mélange de 437 mg de l'amine de la préparation 19 du tableau 2 sous forme de chlorhydrate, et de 750 mg de l'urée de la préparation 1 du tableau 1, sont chauffés dans 15 mL d'éthanol pendant 5 heures. Le milieu réactionnel est évaporé à sec puis repris dans 50 mL de CHCl₃ et 20 mL d'une solution saturée en NaHCO₃. La phase organique est décantée, lavée à l'eau puis par une solution saturée en NaCl. La phase organique est séchée et évaporée sous pression réduite.Le résidu est purifié par chromatographie éclair avec un gradient de 0 à 20% (v/v) d'AcOEt dans le chloroforme. On obtient 275 mg d'un solide jaune. MH⁺: 572.

### EXEMPLE 3 : (Composé N° 4) N-[6-(2,6-dichlorophényl)-2-[(2-méthyl-6-benzoxazolyl)amino]pyrido[2,3-d]pyrimidin-7-yl]-N'-(1,1-diméthyléthyl)-urée.

210 mg de l'amine de la préparation 20 du tableau 2 et 562 mg de l'urée de la préparation 1 du tableau 1, sont chauffés pendant 8 heures à 45°C dans 20 mL d'éthanol contenant 0,02 mL d'HCl concentré. Après concentration sous pression réduite, le résidu est chromatographié sur gel de silice, éluant CHCl₃/MeOH : 98/2 (v/v). 300 mg de produit sous la forme d'un solide jaune sont isolés. MH⁺: 536.

### EXEMPLE 4 : (Composé N° 5) N-[6-(2,6-dichlorophényl)-2-[[2-[(diéthylamino)méthyl]-5-benzofuranyl]amino]pyrido[2,3-d]pyrimidin-7-yl]-N'-(1,1-diméthyléthyl)-urée.

Le composé 5 est préparé de la même manière que le composé 4, au départ de l'amine 21.4 de la préparation 21 du tableau 2.

### EXEMPLE 5 : (Composé N° 6) [[5-[[6-(2,6-dichlorophényl)-7-[[[(1,1-diméthyléthyl)amino]carbonyl]amino]pyrido[2,3-d]pyrimidin-2-yl]amino]-2-benzoxazolyl]méthyl]-carbamate de 1,1-diméthyléthyle.

Le composé 6 est préparé de la même manière que le composé 4, au départ de l'amine 22.4 de la préparation 22 du tableau 2.

### EXEMPLE 6 : (Composé N° 7) N-[2-[[2-(aminométhyl)-5-benzoxazolyl]amino]-6-(2,6-dichlorophényl)pyrido[2,3-d]pyrimidin-7-yl]-N'-(1,1-diméthyléthyl)-urée.

190 mg du composé 6 sont traités pendant 1 heure par 3 mL de TFA dans 2 mL de DCM. Après concentration sous pression réduite, le résidu est repris dans un mélange DCM / eau puis le pH est amené à 9 par ajout d'une solution de Na₂CO₃ à 15%. Après décantation, la phase organique est lavée par de l'eau, puis par une solution saturée en NaCl, séchée, et concentrée sous pression réduite. Le brut est purifié par chromatographie éclair sur gel de silice, éluant 0 à 10% (v/v) de méthanol dans le DCM. 100 mg de solide jaune sont isolés. MH⁺: 551.

### EXEMPLE 7 : (Composé N° 8) N-[6-(2,6-dichlorophényl)-2-(imidazo[1,2-a]pyridin-6-ylamino)pyrido[2,3-d]pyrimidin-7-yl]-N'-(1,1-diméthyléthyl)-urée.

Le composé 8 est préparé de la même manière que le composé 4, au départ de l'amine 23.2 de la préparation 23 du tableau 2.

### EXEMPLE 8 : (Composé N° 9) N-[6-(2,6-dichlorophényl)-2-([1,2,4]triazolo[1,5-a]pyridin-6-ylamino)pyrido[2,3-d]pyrimidin-7-yl]-N'-(1,1-diméthyléthyl)-urée.

Le composé 9 est préparé de la même manière que le composé 4, au départ de l'amine de la préparation 24 du tableau 2.

### EXEMPLE 9 : (Composé N° 10) N-[2-(2,1,3-benzoxadiazol-5-ylamino)-6-(2,6-dichlorophényl)pyrido[2,3-d]pyrimidin-7-yl]-N'-(1,1-diméthyléthyl)-urée.

A un mélange de 0,468 g du produit de la préparation 1 et 0,202 g de l'amine (III) de la préparation 25 du tableau 2, dans 6 mL de DMSO, 0,168 g de tBuOK sont ajoutés en 25 mn puis encore 0,168 g en 1 heure. Après 2 heures d'agitation le milieu réactionnel est extrait par de l'acétate d'éthyle qui est lavé successivement par de l'eau et une solution saturée de NaCl. Après séchage sur Na2SO4 et évaporation de l'acétate d'éthyle, le produit brut est purifié par chromatographie éclair sur gel de silice avec un gradient de 0 à 8% (v/v) d'acétate d'éthyle dans le dichlorométhane. On obtient une poudre beige, m= 0,22 g. MH+=523.

### EXEMPLE 10 : (Composé N° 11) N-[2-(1,3-dihydro-2,2-dioxido-2,1,3-benzothiadiazol-5-ylamino)-6-(2,6-dichlorophényl)pyrido[2,3-d]pyrimidin-7-yl]-N'-(1,1-diméthyléthyl)-urée.

Le composé 11 est préparé de la même manière que le composé 4, au départ de l'amine de la préparation 26 du tableau 2.

Le tableau 3 illustre les structures chimiques et les propriétés physiques de quelques exemples selon l'invention. Dans ces tableaux, Me, Et, iPr et tBu représentent respectivement les groupes méthyle, éthyle, isopropyle et *tert*-butyle, et Boc (ou BOC) représente le groupe *tert*-butoxycarbonyle.

**TABLEAU 3**

| | | |
|---|---|---|
| 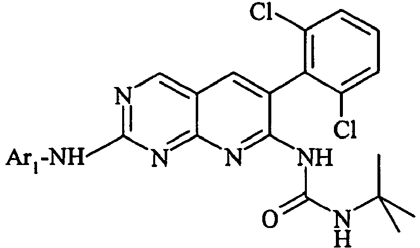 | | (I) |

| Composés | Ar₁ | Caractérisation RMN |
|---|---|---|
| 2 | 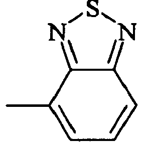 | 1,40 ppm : s: 9H ; 7,45 - 7,80 ppm : m : 5H; 8,15 ppm : s : 1H; 8,35 ppm : s : 1H ; 8,80 ppm : d : 1H ; 9,20 ppm : s : 1H ; 9,55 pmm : s : 1H ; 10,60 ppm : s : 1H. |
| 3 | 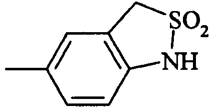 | 1,45 ppm : s: 9H ; 4,45 ppm : s : 2H ; 6,75 ppm : d : 1H ; 7,45 - 7,70 ppm : m : 3H ; 7,80 ppm : de : 1H ; 8,05 ppm : s : 1H ; 8,15 ppm : se : 2H ; 9,05 ppm : s : 1H ; 10,10 ppm : s : 1H ; 10,20 - 10,30 ppm : se : 1H ; 10,60 ppm : s : 1H. |
| 4 | 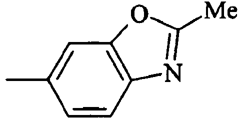 | 1,45 ppm : s: 9H; 2,60 ppm : s : 3H; 7,50 - 7,70 ppm : m : 5H ; 8,10 ppm : s : 1H ; 8,30 ppm : se : 1H ; 8,90 ppm : se : 1H ; 9,15 ppm : s : 1H ; 10,45 ppm : s : 1H ; 10,75 ppm : s : 1H. |
| 5 | 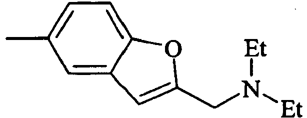 | 1,00 ppm : t : 6H ; 1,45 ppm : s: 9H ; 2,50 ppm : qd : 4H ; 3,70 ppm : s : 2H ; 6,55 ppm : s : 1H ; 7,40 - 7,70 ppm : m : 5H ; 8,10 ppm : s : 2H ; 8,55 ppm : se : 1H ; 9,10 ppm : s : 1H ; 10,15 ppm : s : 1H ; 10,65 ppm: s: 1H. |
| 6 | 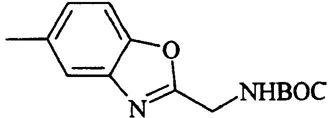 | 1,30 ppm : s : 9H ; 1,40 ppm : s: 9H ; 4,35 ppm : d : 2H ; 7,45 - 7,60 ppm : m : 5H ; 7,75 ppm : dd : 1H ; 8,05 ppm : s : 1H ; 8,10 ppm : se : 1H ; 8,65 ppm : s : 1H ; 9,10 ppm : s : 1H ; 10,30 ppm : s : 1H ; 10,60 ppm : s : 1H. |
| 7 | 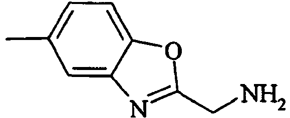 | 1,45 ppm : s: 9H ; 2,10 ppm : se : 2H ; 3,90 ppm : s : 2H ; 7,45 - 7,70 ppm : m : 4H ; 7,80 ppm : dd : 1H ; 8,05 ppm : s : 1H ; 8,15 ppm : se : 1H ; 8,65 ppm : s : 1H ; 9,10 ppm : s : 1H ; 10,25 ppm : s : 1H ; 10,70 ppm : s : 1H. |
| 8 | 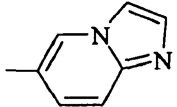 | 1,50 ppm : s: 9H ; 7,40 - 7,70 ppm : m : 7H ; 8,10 ppm : s : 1H ; 8,20 ppm : s : 1H ; 9,10 ppm : s : 1H ; 9,65 ppm : s : 1H ; 10,30 ppm : s : 1 H ; 10,60 ppm : s : 1H. |
| 9 | 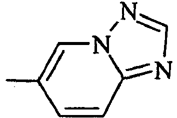 | 1,50 ppm : s: 9H ; 7,45 - 7,70 ppm : m : 3H ; 7,85 ppm : s : 2H ; 8,15 ppm : s : 1H ; 8,25 ppm : d : 1H ; 8,40 ppm : s : 1H ; 9,15 ppm : s : 1H ; 10,10 pmm : s : 1H ; 10,55 ppm : s : 1H ; 10,60 ppm : s : 1H. |
| 10 | 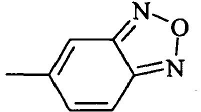 | 1,45 ppm: s: 9H; 7,50-7,70 ppm: mt: 3H; 7,75 ppm: d: 1H; 8,00 ppm: d: 1H; 8,20 ppm: s: 1H; 8,40 ppm: s: 1H; 9,15 ppm: s: 1H; 9,25 ppm: s: 1H; 10,65 ppm: s: 1H; 10,90 ppm: s: 1H. |
| 11 | 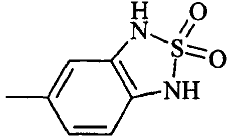 | 1,45 ppm: s: 9H; 6,65 ppm: d: 1H; 7,40 ppm: s: 1H; 7,45-7,70 ppm: m: 4H; 8,05 ppm: s: 2H |
| | | 9,05 ppm: s: 1H; 10,00 ppm: s: 1H; 10,55 ppm: s: 1H; 10,75 ppm: se: 2H. |

Les composés selon l'invention ont fait l'objet d'essais pharmacologiques permettant de déterminer leur activité anticancéreuse.

Les composés de formule (I) selon la présente invention ont été testés in vitro sur un panel de lignées tumorales d'origine humaine provenant :
- de cancer du sein: MDA-MB231 (American Type culture collection, Rockville, Maryland, USA, ATCC-HTB26), MDA-A1 ou MDA-ADR (dite lignée multi-drug resistant MDR, et décrite par E.Collomb et al., dans Cytometry, 12(1):15-25, 1991), et MCF7 (ATCC-HTB22),
- de cancer de la prostate: DU145 (ATCC-HTB81) et PC3 (ATCC-CRL1435),
- de cancer du colon: HCT116 (ATCC-CCL247) et HCT15 (ATCC-CCL225),
- de cancer du poumon: H460 (décrite par Carmichael dans Cancer Research 47 (4):936-942, 1987 et délivré par le National Cancer Institute, Frederick Cancer Research and Development Center, Frederick, Maryland, USA),
- de glioblastome : SF268 (décrite par Westphal dans Biochemical & Biophysical Research Communications 132 (1): 284-289, 1985 et délivré par le National Cancer institute, Frederick Cancer Research and Development Center, Frederick, Maryland, USA),
- de leucémie : CMLT1 (décrite par Kuriyama et al. dans Blood, 74: 1989, 1381-1387, par Soda et al. dans British Journal of Haematology, 59: 1985, 671-679 et par Drexler, dans Leukemia Research, 18: 1994, 919-927 et délivré par la société DSMZ (Deutsche Sammlung von Mikroorganismen und Zellkulturen GmbH) Mascheroder Weg 1b, 38124 Braunschweig, Germany), K-562 (décrite par Lozzio et al., J Natl Cancer Inst 50: 535 (1973), par Lozzio et al., Blood 45: 321 (1975), et délivré par DSMZ n° ACC 10), KG-1a (décrite par Koeffler et al., Blood 56: 265 (1980), et délivré par DSMZ n°ACC 421), et Kasumi-1 (décrite par Asou et al., Blood 77: 2031 (1991), et délivré par DSMZ n° ACC 220).

La prolifération et la viabilité cellulaire ont été déterminée dans un test utilisant le 3-(4,5-diméthylthiazol-2-yl)-5-(3-carboxyméthoxyphényl)-2-(4-sulfophényl)-2*H-*tétrazolium (MTS) selon Fujishita T. et al., Oncology, 2003, 64 (4), 399-406. Dans ce test, on mesure la capacité mitochondriale des cellules vivantes à transformer le MTS en un composé coloré après 72 heures d'incubation d'un composé de formule (I) selon l'invention. Les concentrations en composé selon l'invention, qui conduisent à 50 % de perte de prolifération et de viabilité cellulaire (CI₅₀) sont comprises entre 1 nM et 10 µM, selon la lignée tumorale et le composé testé. Sur la lignée K-562, le composé n°5 présente une CI₅₀ de 5 nM, le composé n°9 présente une CI₅₀ de 19 nM. Sur la lignée SF268, le composé n°7 présente une CI₅₀ de 43 nM.

Ainsi, selon la présente invention, il apparaît que les composés de formule (I) entraînent une perte de prolifération et de viabilité des cellules tumorales. Il apparaît donc que les composés selon l'invention ont une activité anticancéreuse et une activité dans le traitement des autres maladies prolifératives telles que le psoriasis, la resténose, l'arthérosclérose, le SIDA par exemple, ainsi que dans les maladies provoquées par la prolifération des cellules du muscle lisse vasculaire et dans la polyarthrite rhumatoïde.

Ainsi selon un autre de ses aspects, l'invention a pour objet des médicaments qui comprennent un composé de formule (I), ou un sel d'addition de ce dernier à un acide pharmaceutiquement acceptable ou encore un hydrate ou un solvate du composé de formule (I).

Ces médicaments trouvent leur emploi en thérapeutique, notamment dans le traitement ou la prévention des maladies causées ou exacerbées par la prolifération des cellules et en particulier des cellules tumorales. Un produit conforme à l'invention pourra être utilisé pour la fabrication d'un médicament utile pour traiter un état pathologique, en particulier un cancer.

Comme inhibiteur de la prolifération des cellules tumorales, ces composés sont utiles dans la prévention et le traitement des leucémies, des tumeurs solides à la fois primaires et métastatiques, des carcinomes et cancers, en particulier : cancer du sein ; cancer du poumon ; cancer de l'intestin grêle, cancer du colon et du rectum ; cancer des voies respiratoires, de l'oropharynx et de l'hypopharynx ; cancer de l'oesophage ; cancer du foie, cancer de l'estomac, cancer des canaux biliaires, cancer de la vésicule biliaire, cancer du pancréas ; cancers des voies urinaires y compris rein, urothelium et vessie; cancers du tractus génital féminin y compris cancer de l'utérus, du col de l'utérus, des ovaires, chloriocarcinome et trophoblastome; cancers du tractus génital masculin y compris cancer de la prostate, des vésicules séminales, des testicules, tumeurs des cellules germinales; cancers des glandes endocrines y compris cancer de la thyroïde, de l'hypophyse, des glandes surrénales ; cancers de la peau y compris hémangiomes, mélanomes, sarcomes, incluant le sarcome de Kaposi ; tumeurs du cerveau, des nerfs, des yeux, des méninges, incluant astrocytomes, gliomes, glioblastomes, rétinoblastomes, neurinomes, neuroblastomes, schwannomes, méningiomes ; tumeurs malignes hématopoïétiques ; leucémies, (Acute Lymphocytic Leukemia (ALL), Acute Myeloid Leukemia (AML), Chronic Myeloid Leukemia (CML), Chronic lymphocytic leukemia (CLL)) chloromes, plasmocytomes, leucémies des cellules T ou B, lymphomes non hodgkiniens ou hodgkiniens, myélomes, hémopathies malignes diverses.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvate dudit composé, ainsi qu'au moins un excipient pharmaceutiquement acceptable. Une composition pharmaceutique peut aussi contenir en outre un autre principe anticancéreux.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvate ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Les composés de formule (I) ci-dessus peuvent être utilisés à des doses journalières de 0,002 à 2000 mg par kilogramme de poids corporel du mammifère à traiter, de préférence à des doses journalières de 0,1 à 300 mg/kg. Chez l'être humain, la dose peut varier de préférence de 0,02 à 10000 mg par jour, plus particulièrement de 1 à 3000 mg selon l'âge du sujet à traiter ou le type de traitement : prophylactique ou curatif.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvates.

Selon la présente invention, le ou les composés de formule (I) peuvent être administrés en association avec un (ou plusieurs) principe(s) actif(s) anticancéreux, en particulier des composés antitumoraux tels que les agents alkylants tels que les alkylsulfonates (busulfan), la dacarbazine, la procarbazine, les moutardes azotées (chlorméthine, melphalan, chlorambucil), cyclophosphamide, ifosfamide; les nitrosourées tels que la carmustine, la lomustine, la sémustine, la streptozocine; les alcaloïdes antinéoplasiques tels que la vincristine, la vinblastine ; les taxanes tel que le paclitaxel ou le taxotère ; les antibiotiques antinéoplasiques tels que l'actinomycine; les agents intercalants, les antimétabolites antinéoplasiques, les antagonistes des folates, le méthotrexate; les inhibiteurs de la synthèse des purines; les analogues de la purine tels que mercaptopurine, 6-thioguanine; les inhibiteurs de la synthèse des pyrimidines, les inhibiteurs d'aromatase, la capécitabine, les analogues de la pyrimidine tels que fluorouracil, gemcitabine, cytarabine et cytosine arabinoside; le bréquinar ; les inhibiteurs de topoisomérases tels que la camptothécine ou l'étoposide ; les agonistes et antagonistes hormonaux anticancéreux incluant le tamoxifène; les inhibiteurs de kinase, l'imatinib; les inhibiteurs de facteurs de croissance; les antiinflammatoires tels que le pentosane polysulfate, les corticostéroïdes, la prednisone, la dexamethasone; les antitopoisomérases tels que l'étoposide, les antracyclines incluant la doxorubicine, la bléomycine, la mitomycine et la méthramycine; les complexes métalliques anticancéreux, les complexes du platine, le cisplatine, le carboplatine, l'oxaliplatine ; l'interféron alpha, le triphénylthiophosphoramide, l'altrétamine ; les agents antiangiogéniques ; la thalidomide ; les adjuvants d'immunothérapie ; les vaccins.

Selon la présente invention les composés de formule (I) peuvent également être administrés en association avec un ou plusieurs autres principes actifs utiles dans une des pathologies indiquées ci-dessus, par exemple un agent anti-émétique, anti-douleur, anti-inflammatoire, anti-cachexie.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

## Revendications

1. Composé répondant à la formule (I) : 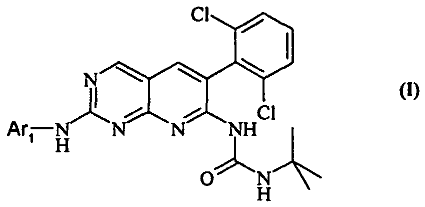 **caractérisé en ce que** Ar₁ est choisi parmi :

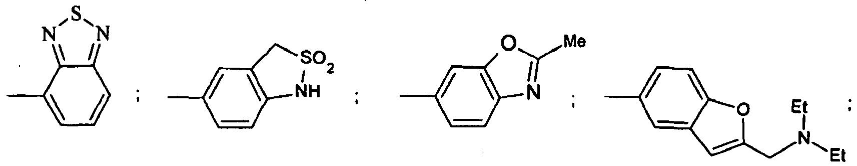

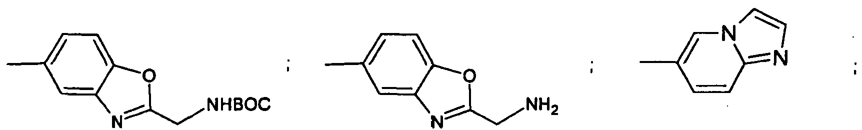

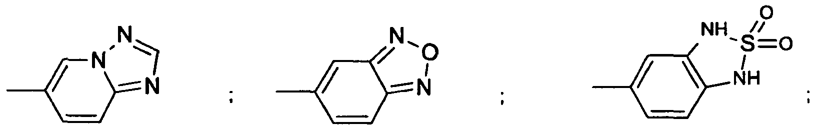

2. Composé selon la revendication 1 **caractérisé en ce qu'**il est sous forme :
non chirale, ou
racémique, ou
enrichie en un stéréo-isomère, ou
enrichie en un énantiomère ;
**en ce qu'**il est éventuellement salifié,
et **en ce qu'**il est éventuellement hydraté ou solvaté.

3. Composition pharmaceutique, **caractérisée en ce qu'**elle comprend un composé selon la revendication 1 ou 2, ainsi qu'au moins un excipient pharmaceutiquement acceptable.

4. Composition pharmaceutique selon la revendication 3, **caractérisée en ce qu'**elle contient en outre au moins un autre principe actif anticancéreux.

5. Médicament, **caractérisé en ce qu'**il comprend un composé selon la revendication 1 ou 2 ou un sel d'addition de ce composé à un acide pharmaceutiquement acceptable, ou encore un hydrate ou un solvate du composé de formule (I).

6. Utilisation d'un composé selon la revendication 1 ou 2 pour la préparation d'un médicament destiné au traitement et à la prévention de maladies causées ou exacerbées par la prolifération des cellules.

7. Utilisation selon la revendication 6, pour la prévention et le traitement des leucémies, des tumeurs solides primaires et métastatiques, des carcinomes et cancers.

8. Procédé de préparation d'un composé selon la revendication 1 ou 2, **caractérisé en ce que** l'on fait réagir sur un composé de formule : 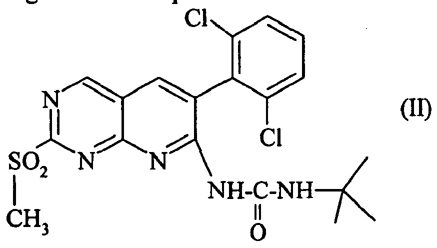 , une amine de formule Ar'₁NH₂ (III) dans laquelle Ar'₁ représente Ar₁, tel que défini à la revendication 1 ou 2 ou un précurseur de Ar₁ ; le cas échéant on transforme le groupe Ar'₁ du composé ainsi obtenu en un groupe Ar₁.

9. Procédé de préparation d'un composé selon la revendication 1 ou 2, **caractérisé en ce que** l'on fait réagir :
(i) un composé de formule : 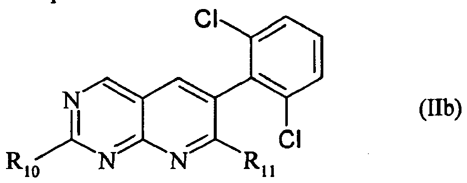 dans laquelle R₁₀ est un groupe partant tel que : (a) halogène, en particulier Cl ou Br, ou (b) alkyl-S(O)m- dans lequel m = 0, 1, ou 2 ; dans laquelle R₁₁ est NHC(O)-NH-tBu; et
(ii) une amine de formule Ar'₁NH₂ (III) dans laquelle Ar'₁ représente Ar₁ tel que défini pour (I) ou un précurseur de Ar₁ ; le cas échéant on transforme le groupe Ar'₁ du composé ainsi obtenu en un groupe Ar₁ ;
dans lequel :
(a) lorsque R₁₀ est halogène ou alkyl-S(O)ₘ- avec m = 2, la réaction est effectuée dans un solvant à une température comprise entre la température ambiante et la température de reflux du solvant ;
(b) lorsque R₁₀ est alkyl-S(O)ₘ- avec m = 0 ou 1, la réaction est effectuée avec Ar' ₁NH₂ (III) à l'état fondu :
le cas échéant, les fonctions amines présentes dans le groupe Ar'₁ du composé (III) sont préalablement salifiées ou protégées.

10. Procédé selon la revendication 9 dans lequel le solvant est polaire.

11. Procédé selon la revendication 10 dans lequel le solvant est
(i) le tétrahydrodurane, le diméthylsulfoxide ou l'éthanol, utilisé éventuellement en présence d'une trace d'acide tel que l'acide chlorhydrique ; ou
(ii) le diméthylsulfoxide utilisé en présence d'une base forte telle que tBuOK ;

## Patentansprüche

1. Verbindung entsprechend der Formel (I): 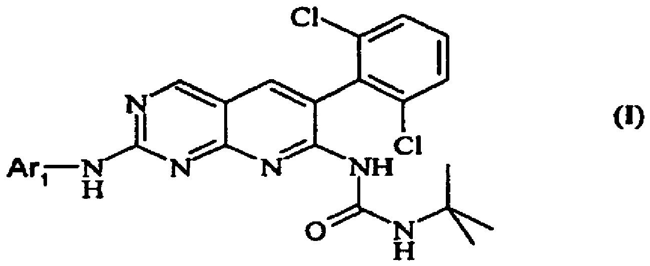 **dadurch gekennzeichnet, dass** Ar₁ ausgewählt wird aus:

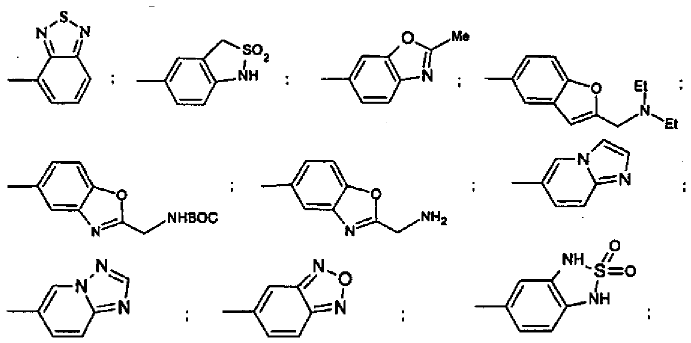

2. Verbindung nach Anspruch 1, **dadurch gekennzeichnet, dass** sie in der Form vorliegt:
achiral oder
racemisch oder
bereichert um ein Stereoisomer oder
bereichert um ein Enantiomer;
dass sie gegebenenfalls in ein Salz überführt ist,
und dass sie gegebenenfalls hydriert oder solvatisiert ist.

3. Pharmazeutische Zusammensetzung, **dadurch gekennzeichnet, dass** sie eine Verbindung nach den Ansprüchen 1 oder 2 sowie mindestens einen pharmazeutischen Träger umfasst.

4. Pharmazeutische Zusammensetzung nach Anspruch 3, **dadurch gekennzeichnet, dass** sie außerdem mindestens einen antikanzerogenen aktiven Inhaltsstoff enthält.

5. Medikament, **dadurch gekennzeichnet, dass** es eine Verbindung nach Anspruch 1 oder Anspruch 2 oder ein Additionssalz dieser Verbindung zu einer pharmazeutisch akzeptablen Säure oder auch ein Hydrat oder ein Solvat der Verbindung der Formel (I) umfasst.

6. Verwendung einer Verbindung nach Anspruch 1 oder Anspruch 2 für die Herstellung eines Medikaments, das zur Behandlung und Prävention von Krankheiten dient, die durch die Proliferation von Zellen verursacht oder verschlechtert werden.

7. Verwendung nach Anspruch 6 zur Prävention und Behandlung von Leukämie, von primären und metastatischen soliden Tumoren, von Karzinomen und Krebserkrankungen.

8. Verfahren zur Herstellung einer Verbindung nach Anspruch 1 oder Anspruch 2, **dadurch gekennzeichnet, dass** man mit einer Verbindung der Formel: 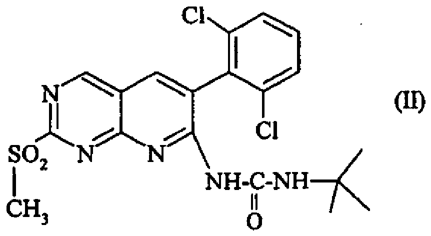 ein Amin der der Formel Ar'₁NH₂ (III) reagieren lässt, wobei Ar'₁ Ar₁, wie es in Anspruch 1 oder Anspruch 2 definiert ist, oder einen Precursor von Ar₁ darstellt; wobei man gegebenenfalls die so erhaltene Gruppe Ar'₁ der Verbindung in eine Gruppe Ar₁ transformiert.

9. Verfahren zur Herstellung einer Verbindung nach Anspruch 1 oder Anspruch 2, **dadurch gekennzeichnet, dass** man reagieren lässt:
(i) eine Verbindung der Formel: 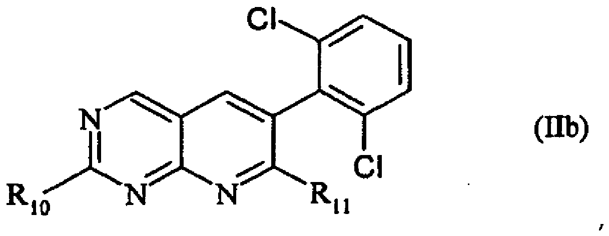 in der R₁₀ eine Abgangsgruppe ist wie: (a) Halogen, insbesondere Cl oder Br, oder (b) Alkyl-S(O)m-, bei dem m = 0, 1 oder 2 ist; in der R₁₁ NHC(O)-NH-tBu ist; und
(ii) ein Amin der Formel Ar'₁NH₂ (III), bei der Ar'₁ Ar₁, wie es für (I) definiert ist, oder einen Precursor von Ar₁ darstellt; wobei man gegebenenfalls die so erhaltene Gruppe Ar'₁ der Verbindung in eine Gruppe Ar₁ transformiert, bei der:
(a) wenn R₁₀ ein Halogen oder ein Alkyl-S(O)m- mit m = 2 ist, die Reaktion in einem Lösungsmittel bei einer Temperatur zwischen der Umgebungstemperatur und der Rückflusstemperatur des Lösungsmittels durchgeführt wird;
(b) wenn R₁₀ ein Alkyl-S(O)m- mit m = 0 oder 1 ist, die Reaktion mit Ar'₁NH₂ (III) in geschmolzenem Zustand durchgeführt wird;
wobei gegebenenfalls die in der Gruppe Ar'₁ der Verbindung (III) vorhandenen Aminfunktionen vorher in ein Salz überführt oder geschützt werden.

10. Verfahren nach Anspruch 9, bei dem das Lösungsmittel polar ist.

11. Verfahren nach Anspruch 10, bei dem das Lösungsmittel
(i) Tetrahydroduran, Dimethylsulfoxid oder Ethanol ist, gegebenenfalls verwendet in Gegenwart einer Säurespur, wie Chlorwasserstoffsäure; oder
(ii) Dimethylsulfoxid ist, verwendet in Gegenwart einer starken Base, wie tBuOK.

## Claims

1. Compound corresponding to the formula (I) : 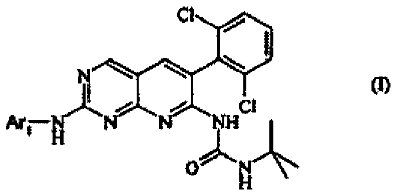 **characterized in that** Ar₁ is chosen from:

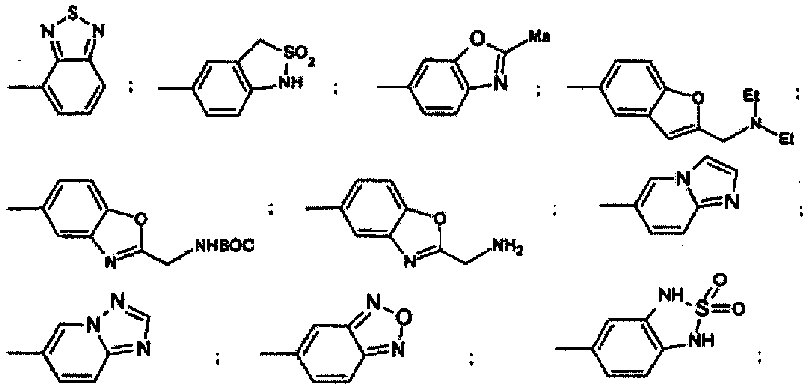

2. Compound according to claim 1, **characterized in that** it is in a form which is:
non-chiral, or
racemic, or
enriched in one stereoisomer, or
enriched in one enantiomer;
**in that** it is optionally in salt form,
and **in that** it is optionally hydrated or solvated.

3. Pharmaceutical composition, **characterized in that** it comprises a compound according to claim 1 or 2, as well as at least one pharmaceutically acceptable excipient.

4. Pharmaceutical composition according to claim 3, **characterized in that** it also comprises at least one other anticancer active principle.

5. Medicament, **characterized in that** it comprises a compound according to claim 1 or 2 or an addition salt of this compound with a pharmaceutically acceptable acid, or also a hydrate or a solvate of the compound of the formula (I).

6. Use of a compound according to claim 1 or 2 for the preparation of a medicament for the treatment and the prevention of diseases caused or exacerbated by the proliferation of cells.

7. Use according to claim 6 for the prevention and the treatment of leukaemias, primary and metastased solid tumours, carcinomas and cancers.

8. Process for the preparation of a compound according to claim 1 or 2, **characterized in that** a compound of the formula: 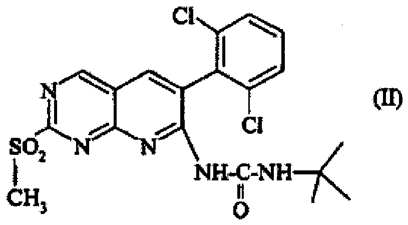 is reacted with an amine of the formula Ar'₁NH₂ (III), in which Ar'₁ represents Ar₁ as defined in claim 1 or 2 or a precursor of Ar₁; and where appropriate the group Ar'₁ of the compound obtained in this way is converted into a group Ar₁.

9. Process for the preparation of a compound according to claim 1 or 2, **characterized in that**:
(i) a compound of the formula: 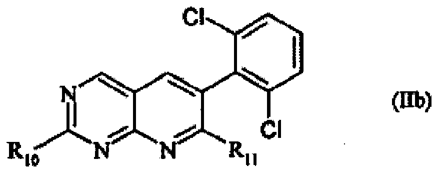 in which R₁₀ is a leaving group such as: (a) halogen, in particular Cl or Br, or (b) alkyl-S(O)ₘ-, in which m = 0, 1 or 2; in which R₁₁ is NHC(O)-NH-tBu; and
(ii) an amine of the formula Ar'₁NH₂ (III), in which Ar'₁ represents Ar₁ as defined for (I) or a precursor of Ar₁, are reacted; where appropriate the group Ar'₁ of the compound obtained in this way is converted into a group Ar₁;
in which:
(a) if R₁₀ is halogen or alkyl-S(O)ₘ-, where m = 2, the reaction is carried out in a solvent at a temperature between ambient temperature and the reflux temperature of the solvent;
(b) if R₁₀ is alkyl-S(O)ₘ-, where m = 0 or 1, the reaction is carried out with Ar'₁NH₂ (III) in the molten state;
where appropriate the amine functions present in the group Ar'₁ of compound (III) are converted into salt form or protected beforehand.

10. Process according to claim 9, in which the solvent is polar.

11. Process according to claim 10, in which the solvent is
(i) tetrahydrofuran, dimethylsulfoxide or ethanol, optionally used in the presence of a trace of acid, such as hydrochloric acid; or
(ii) dimethylsulfoxide used in the presence of a strong base, such as tBuOK.
